# FASCICULE DE BREVET EUROPEEN

(11) **EP 0 443 963 B1**
(45) Date de publication et mention de la délivrance du brevet: **13.04.1994**
(21) Numéro de dépôt: 91420031.6
(22) Date de dépôt: 01.02.1991
(51) Int. Cl.: B01F 15/00, G01P 13/00, A61M 1/36

(54) **Détecteur d'écoulement d'un premier liquide dans un circuit de circulation d'un second liquide**
Detektor für den Abfluss einer ersten Flüssigkeit im Kreislauf einer zweiten Flüssigkeit
Detector for detection of the flow of a first liquid in a circulation of a second liquid

(30) Priorité: 23.02.1990 FR 9002548
(43) Date de publication de la demande: 28.08.1991
(73) Titulaire: HOSPAL INDUSTRIE, F-69883 Meyzieu Cédex (FR)
(72) Inventeur: Chevallet, Jacques, F-69360 Serezin du Rhone (FR)

(56) Documents cités:
- EP-A- 0 219 800
- WO-A-89/12228
- US-A- 3 456 648
- IBM TECHNICAL DISCLOSURE BULLETIN, vol. 20, no. 4, septembre 1977, pages 1442-1443, New York, US; J.C. ARNHART et al.: "Flow interrupt detector"

## Description

La présente invention a pour objet un détecteur d'écoulement d'un premier liquide dans un circuit de circulation d'un second liquide, en particulier un détecteur d'écoulement d'un liquide médical dans un circuit de circulation extracorporelle de sang.

Cette invention trouve application dans les techniques de traitement du sang qui mettent en oeuvre une circulation extracorporelle du sang et où il est nécessaire d'injecter au patient un liquide médical (de perfusion ou de substitution), techniques parmi lesquelles on peut citer, par exemple, l'hémofiltration, l'hémodiafiltration ou la plasmaphérèse.

Dans ces techniques de traitement, l'introduction du liquide médical dans le sang se fait généralement au niveau du circuit de circulation extracorporelle du sang, ceci afin d'éviter de multiplier les accès directs au système vasculaire du patient. C'est ainsi que, dans les circuits de liquide médical classiques, la canalisation par laquelle s'écoule le liquide médical à partir d'un réservoir de liquide médical est connectée à une de ses extrémités à une canalisation du circuit sang, en amont ou en aval d'un dispositif de traitement du sang.

Un incident qui se produit assez régulièrement dans les installations de traitement de sang du type mentionné plus haut est l'interruption involontaire de l'écoulement du liquide médical, due par exemple à l'oubli d'un clamp sur une canalisation du circuit de liquide médical ou à l'arrêt d'une pompe de circulation utilisée pour provoquer l'écoulement du liquide médical et en réguler le débit. Cet incident pouvant avoir des conséquences graves pour le patient s'il n'est pas détecté rapidement, deux moyens de détection principaux sont utilisés actuellement, seuls ou en combinaison, pour détecter un arrêt de l'écoulement du liquide médical.

Un premier moyen de détection utilisé sur les circuits de liquide médical comprenant une pompe de circulation est constitué par un détecteur de rotation de la pompe. Ce moyen de détection ne résoud que partiellement le problème posé puisqu'il ne permet de détecter qu'une des causes possibles de l'arrêt de l'écoulement du liquide médical, à savoir l'arrêt de la pompe. En particulier, il ne permet en aucun cas de détecter la présence d'un clamp sur la canalisation du circuit de liquide médical.

Un second moyen de détection utilisé est un détecteur de pression captant la pression régnant dans une chambre de dégazage généralement prévue sur le circuit du liquide médical, soit en amont, soit en aval de la pompe de circulation, quand le circuit en comporte une. Ce moyen de détection n'est pas fiable non plus puisque, selon la localisation d'un clamp sur la canalisation de liquide médical par rapport à la chambre de dégazage et à la pompe, il détecte ou non l'absence d'écoulement du liquide médical : à titre d'exemple, la pose d'un clamp sur la portion de canalisation s'étendant entre la chambre de dégazage et la pompe ne provoque pas de variation de pression sensible dans la chambre de dégazage, que la chambre de dégazage soit disposée en aval ou en amont de la pompe par rapport au sens d'écoulement du liquide médical.

Il faut noter, par ailleurs, que l'insuffisance de ces moyens de détection ne peut être palliée par l'utilisation des détecteurs de débits connus, utilisables sur un circuit de liquide médical : en effet, compte tenu de la spécificité de ce type de circuit, qui est d'être à usage unique et de contenir un liquide stérile qui ne doit être pollué en aucun cas, un détecteur de débit utilisable sur un tel circuit doit n'avoir aucune partie au contact du liquide et, plus généralement, doit être extérieur au circuit de liquide médical. Or, les détecteurs de débit connus de ce type, les détecteurs ultrasoniques par exemple, sont impropres à détecter de façon fiable l'écoulement d'un liquide parfaitement limpide ne véhiculant ni particules, ni microbulles.

La présente invention a pour but de remédier aux lacunes de l'état de la technique et de proposer des moyens permettant de détecter de façon fiable l'écoulement d'un liquide médical dans un circuit de circulation extracorporelle de sang.

Pour atteindre ce but on prévoit, conformément à l'invention, un détecteur d'écoulement d'un premier liquide (un liquide médical, en particulier), prévu pour s'écouler à l'intérieur d'un premier moyen de canalisation, dans un second liquide (du sang, en particulier) prévu pour s'écouler à l'intérieur d'un second moyen de canalisation auquel est connecté le premier moyen de canalisation, le détecteur comprenant des moyens de détection pour détecter la présence du second liquide dans le premier moyen de canalisation à proximité de sa connexion au second moyen de canalisation.

Ce détecteur est parfaitement fiable en ce qu'il permet de contrôler non pas seulement l'existence d'un débit dans la canalisation de liquide médical, mais l'écoulement effectif du liquide médical dans le sang.

Selon un premier mode de réalisation de l'invention, les premier et second moyens de canalisation sont constitués par des tubes au niveau de leur connexion.

Ce détecteur d'écoulement présente l'intérêt d'être très facilement réalisable et à bon marché. Il présente aussi l'intérêt que, par une simple modification de sa position dans l'espace, correspondant à une modification de l'inclinaison, par rapport à l'horizontale, de la partie terminale de première canalisation, on peut régler le débit de seuil au-dessous duquel il déclenche.

Selon un second mode de réalisation de l'invention, le second moyen de canalisation comprend une chambre et le premier moyen de canalisation est connecté à la chambre à un niveau prévu pour être baigné en permanence par le second liquide.

Conformément à l'invention, on prévoit aussi un dispositif de détection à seuil de détection réglable, comportant un détecteur selon le premier mode de réalisation mentionné ci-dessus fixé à une platine comprenant des moyens pour maintenir les tubes relativement aux moyens de détection du second liquide, la platine étant montée pivotante sur un support pour permettre le positionnement du tube du premier moyen de canalisation dans au moins une position allant d'une position horizontale à une une position verticale.

Ce dispositif de détection présente l'intérêt d'avoir un seuil de détection réglable par simple pivotement de la platine (ce seuil correspondant à une vitesse d'écoulement déterminée du liquide médical au niveau de la connexion des canalisation, au-dessous de laquelle du sang pénètre dans la première canalisation).

D'autres caractéristiques et avantages de la présente invention apparaîtront à la lecture de la description qui suit. On se reportera aux dessins annexés sur lesquels :
Les figures 1 à 3 sont des vues en coupe schématiques, selon un plan passant sensiblement par l'axe des canalisations, d'un premier mode de réalisation de l'invention représenté dans trois positions différentes ;
La figure 4 est une vue en élévation schématique d'un détecteur d'écoulement à seuil de détection réglable conforme à l'invention ; et,
La figure 5 est une vue en coupe schématique, selon un plan passant sensiblement par l'axe des canalisations, d'un second mode de réalisation de l'invention.

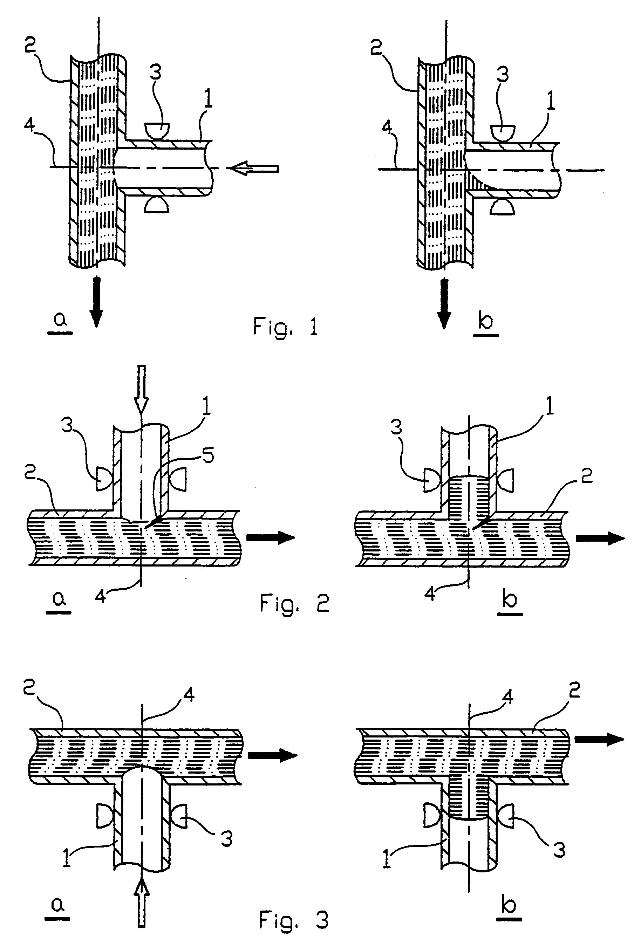

Sur les figures 1 à 3, on a représenté la connexion de la partie terminale, constituée par un tube souple 1, d'un premier moyen de canalisation 1 pour l'écoulement d'un liquide médical à une portion tubulaire 2 d'un second moyen de canalisation faisant partie d'un circuit de circulation extracorporelle du sang. Conformément à l'invention, un premier type de détecteur d'écoulement de liquide médical est constitué par un détecteur de sang 3 disposé sur le tube 1 à proximité du tube 2, pour intercepter sensiblement l'axe 4 de la canalisation 1. La distance entre le détecteur de sang 3 et le tube 2 peut être ajustée en fonction du temps de réponse souhaité du détecteur d'écoulement. Comme cela sera explicité plus loin dans la description du fonctionnement de ce détecteur d'écoulement, si l'on souhaite un temps de réponse minimal, on disposera le détecteur de sang 3 aussi près que possible du tube 2, compte tenu de l'encombrement du détecteur de sang choisi.

Comme détecteur de sang 3, on peut utiliser tout détecteur connu permettant de détecter la présence d'un liquide dans une canalisation, soit, par exemple, un détecteur optique délivrant une information de colorométrie ou de turbidimétrie (auquel cas le tube 1 sera fait dans un matériau transparent), un détecteur inductif ou un détecteur capacitif. En raison de leur fiabilité et de leur faible coût, les détecteurs optiques trouvent une application particulièrement intéressante dans le détecteur selon l'invention.

Le fonctionnement de ce détecteur est fondé sur l'exploitation de deux caractéristiques du sang qui sont, d'une part, sa faculté de sédimenter rapidement quand il n'est pas en situation de coaguler et, d'autre part, sa faculté de diffuser rapidement en milieu aqueux (le sang n'est évidemment pas le seul liquide à posséder l'une et/ou l'autre de ces caractéristiques). Grâce à ces caractéristiques du sang, il n'est pas nécessaire que le tube 1 de liquide médical ait une direction privilégiée dans l'espace pour que le détecteur selon l'invention fonctionne, ainsi que cela apparaît sur les figures 1, 2 et 3.

Sur la figure 1, l'axe 4 du tube 1 est sensiblement horizontal. Le tube 2 étant parcouru par un flux de sang, aussi longtemps que du liquide médical s'écoule dans le tube 1 et arrive, avec une certaine vitesse, au contact du sang (figure 1a), le liquide médical empêche le sang de pénétrer dans le tube 1. Si la vitesse du liquide médical tombe en-dessous d'une certaine valeur, le sang au contact du liquide médical stagnant dans le tube 1 se met à sédimenter quasi instantanément et pénètre dans le tube 1 (figure 1b). Quand il arrive au niveau du détecteur 3, sa présence est détectée, ce qui permet de déclencher une alarme. A titre d'exemple, avec des tubes 1 et 2 ayant un diamètre intérieur d'environ quatre millimètres et demi, le débit du liquide médical au-dessous duquel le sang sédimente est d'environ cinquante millilitres/heure. Dans cette configuration des canalisations, le détecteur 3 étant disposé à environ cinq millimètres de l'extrémité du tube 1, le sang met de l'ordre de cinq secondes pour atteindre le détecteur 3 à partir de l'interruption de l'écoulement du liquide médical.

Sur la figure 2, l'axe 4 du tube 1 du liquide médical est sensiblement vertical et l'arrivée du liquide se fait par le haut. Le raccord des canalisations 1, 2 comprend avantageusement une ailette 5 s'étendant sur une partie de la zone de jonction aval des canalisations (par rapport au sens d'écoulement du sang), cette ailette faisant légèrement saillie en oblique à l'intérieur du tube 2 pour favoriser la déflection, dans le tube 1, d'un filet de sang périphérique de la veine de sang s'écoulant dans le tube 2. Lorsque la vitesse du liquide médical dépasse une valeur de seuil au niveau de la jonction des canalisations, la fonction de déflection de l'ailette 5 est neutralisée (figure 2a). Si la vitesse du liquide médical tombe au-dessous de cette valeur, le liquide médical ne s'oppose plus à la déflection du sang par l'ailette 5 : du sang se répand alors rapidement par convection dans le tube 1, où il est détecté par le détecteur de sang 3 (figure 2b).

Sur la figure 3, l'axe 4 du tube 1 du liquide médical est sensiblement vertical et l'arrivée du liquide se fait par le bas. Le mode de fonctionnement du détecteur d'écoulement de liquide médical dans cette position est le même que dans la position représentée sur la figure 1, à ceci près que la vitesse de seuil du liquide médical au-dessous de laquelle le sang sédimente, toutes choses étant égales par ailleurs, est beaucoup plus importante. A titre de comparaison, avec un détecteur de sang 3 placé sur un tube 1 ayant environ quatre millimètres et demi de diamètre intérieur, à environ cinq millimètres d'un tube 2 ayant également quatre millimètres et demi de diamètre intérieur, comme dans l'exemple donné plus haut en relation à la figure 1, le débit de seuil n'est plus de environ cinquante millilitres/heure mais de environ cinq cents millilitres/heure. Naturellement, à débit de liquide médical égal, il est possible d'abaisser cette valeur de seuil en choisissant un tube 1 ayant un diamètre intérieur inférieur.

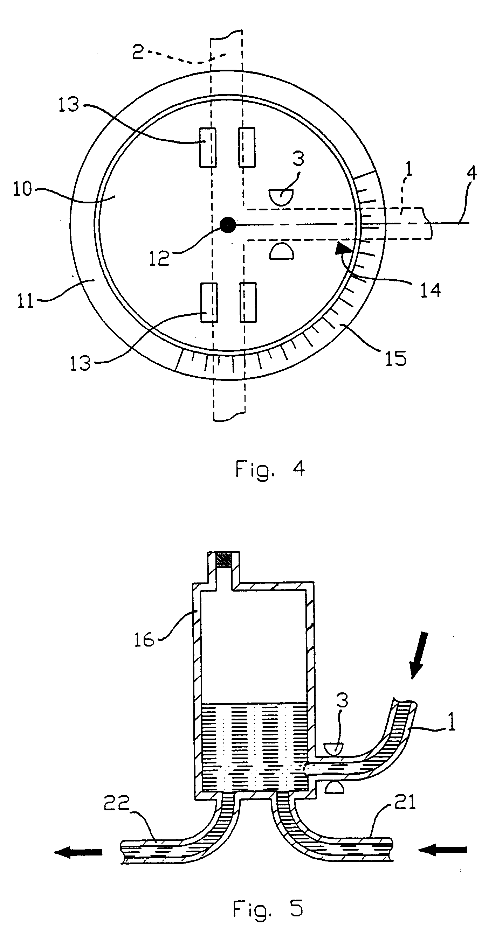

Sur la figure 4, on a représenté un dispositif de détection d'écoulement de liquide médical à seuil de déclenchement réglable. Ce dispositif comporte une platine 10 circulaire montée pivotante sur un support 11, autour d'un axe 12 passant par son centre. Le support 11 est prévu pour être fixé de façon que l'axe 12 soit sensiblement horizontal (ce support peut aussi faire partie intégrante d'un appareil). Un détecteur de sang 3 est fixé sur la platine 10 pour intercepter une direction sensiblement radiale. La platine 10 comprend deux pinces 13 pour maintenir un tube 2 de circuit sang auquel est connecté un tube 1 de circuit de liquide médical, de telle façon que le tube 1 s'étende selon la direction radiale interceptée par le détecteur 3 et que le détecteur 3 soit à proximité du tube 2. Enfin, la platine 10 porte à sa périphérie un index 14 disposé en regard d'un secteur gradué 15, solidaire du socle 11. Sur le mode de réalisation représenté, le secteur gradué s'étend sur un quart de cercle et correspond à des positions d'un tube 1 de circuit de liquide médical allant de l'horizontale à la verticale, le liquide dans ce dernier cas arrivant par le bas. Comme variantes à cette disposition, on peut aussi prévoir un secteur gradué d'un quart de cercle correspondant à des positions du tube 2 allant de l'horizontale à la verticale, le liquide arrivant par le haut, ou encore un secteur gradué d'un demi cercle correspondant à toutes les positions que le tube 2 peut occuper dans un plan vertical.

Les graduations du secteur 15 correspondent avantageusement aux débits de seuil au-dessous desquels, pour un type de canalisation (diamètre intérieur) et de connexion de canalisations donnés (raccordement en T ou en Y, présence ou non d'ailette 5), le dispositif de détection déclenche.

La figure 5 représente second mode de réalisation du détecteur d'écoulement de liquide médical selon l'invention.

Dans cette variante, le second moyen de canalisation, prévu pour la circulation du sang, comprend une chambre 16, qui peut être une chambre de dégazage, à la base de laquelle sont connectés un premier tube souple 21 par lequel le sang pénètre dans la chambre 16 et un second tube souple 22 par lequel le sang quitte la chambre 16. A cette chambre est également connecté le premier moyen de canalisation, dont l'extrémité est constituée par un tube souple 1, prévu pour la circulation d'un liquide médical. Le raccordement du tube 1 à la chambre 16 se fait à un niveau de la chambre qui est toujours baigné par le sang, en fonctionnement normal. Un détecteur de sang 3 est déposé sur le tube 1 à proximité de sa connexion à la chambre 16. La distance entre le détecteur de sang 3 et la chambre 16 peut être ajustée en fonction du temps de réponse souhaité du détecteur d'écoulement.

Ce second mode de réalisation de l'invention fonctionne de la même façon que celui qui a été décrit en relation à la figure 1.

La présente invention n'est pas limitée aux modes de réalisation qui viennent d'être décrits. En particulier, le détecteur selon l'invention peut être utilisé à d'autres fins que de détecter l'écoulement d'un liquide médical dans un circuit de circulation extracorporelle de sang ; il peut ainsi être utilisé, par exemple, dans un dispositif de perfusion pour détecter l'écoulement d'un premier liquide médical dans un second liquide médical circulant dans une canalisation connectée à un patient, les deux liquides ne devant être mélangés que juste avant d'être infusés, l'un des liquides étant transparent et l'autre liquide étant coloré.

## Revendications

1. Détecteur d'écoulement d'un premier liquide, prévu pour s'écouler à l'intérieur d'un premier moyen de canalisation (1), dans un second liquide, prévu pour s'écouler à l'intérieur d'un second moyen de canalisation (2) auquel est connecté le premier moyen de canalisation (1), le détecteur comprenant des moyens de détection (3) pour détecter la présence du second liquide dans le premier moyen de canalisation (1) à proximité de sa connexion au second moyen de canalisation (2).

2. Détecteur selon la revendication 1, caractérisé en ce que les premier et second moyens de canalisation sont constitués par des tubes (1, 2) au niveau de leur connexion.

3. Détecteur selon la revendication 1, caractérisé en ce que le second moyen de canalisation (2) comprend une chambre (16) et en ce que le premier moyen de canalisation (1) est connecté à la chambre (16) à un niveau prévu pour être baigné en permanence par le second liquide.

4. Dispositif de détection à seuil de détection réglable, comportant un détecteur selon la revendication 2 fixé à une platine (10) comprenant des moyens (13) pour maintenir les tubes (1, 2) relativement aux moyens de détection (3), la platine (10) étant montée pivotante sur un support (11) pour permettre le positionnement du tube (1) du premier moyen de canalisation dans au moins une position allant d'une position horizontale à une une position verticale.

5. Détecteur selon une des revendications précédentes, caractérisé en ce que les moyens de détection (3) sont des moyens de détection optiques.

6. Détecteur selon une des revendications précédentes, caractérisé en ce qu'il est utilisé pour la détection de l'écoulement d'un liquide médical dans un circuit de circulation extracorporelle de sang.

## Patentansprüche

1. Detektor für den Abfluß einer ersten Flüssigkeit, die zum Abfluß in das Innere einer ersten Leitungseinrichtung (1) vorgesehen ist, in eine zweite Flüssigkeit, die zum Abfluß in das Innere einer zweiten Leitungseinrichtung (2) vorgesehen ist, an die die erste Leitungseinrichtung (1) angeschlossen ist, wobei der Detektor Erfassungsmittel (3) zum Erfassen der Anwesenheit der zweiten Flüssigkeit in der ersten Leitungseinrichtung (1) in der Nähe ihres Anschlusses an die zweite Leitungseinrichtung (2) aufweist.

2. Detektor gemäß Anspruch 1, dadurch gekennzeichnet, daß die erste und zweite Leitungseinrichtung von Rohren (1, 2) gebildet sind.

3. Detektor gemäß Anspruch 1, dadurch gekennzeichnet, daß die zweite Leitungseinrichtung (2) eine Kammer (16) aufweist, und dadurch, daß die erste Leitungseinrichtung (1) an die Kammer (16) auf einer Höhe angeschlossen ist, die vorgesehen ist, um permanent von der zweiten Flüssigkeit gefüllt zu sein.

4. Erfassungsvorrichtung mit einstellbarer Erfassungsschwelle, die einen Detektor gemäß Anspruch 2 aufweist, der an einer Halteplatte (10) befestigt ist, die Mittel (13) zum Halten der Rohe (1, 2) gegenüber den Erfassungsmitteln (3) aufweist, wobei die Halteplatte (10) schwenkbar auf einem Träger (11) angebracht ist, um die Positionierung des Rohrs (1) der ersten Leitungseinrichtung in zumindest einer von einer horizontalen Position in eine vertikale Position übergehenden Position zu ermöglichen.

5. Detektor gemäß einem der vorherigen Ansprüche, dadurch gekennzeichnet, daß die Erfassungsmittel (3) optische Erfassungsmittel sind.

6. Detektor gemäß einem der vorherigen Ansprüche, dadurch gekennzeichnet, daß er benutzt wird zur Erfassung des Abflusses einer medizinischen Flüssigkeit in einen außerkörperlichen Blutkreislauf.

## Claims

1. Detector for detection of the flow of a first liquid, intended to flow inside a first channel means (1), in a second liquid, intended to flow inside a second channel means (2) to which the first channel means (1) is connected, the detector comprising detection means (3) for detecting the presence of the second liquid in the first channel means (1) in proximity to its connection to the second channel means (2).

2. Detector according to Claim 1, characterized in that the first and second channel means consist of tubes (1, 2) at the level of their connection.

3. Detector according to Claim 1, characterized in that the second channel means (2) comprises a chamber (16), and in that the first channel means (1) is connected to the chamber (16) at a level intended to be filled permanently by the second liquid.

4. Detection device having an adjustable detection threshold, comprising a detector according to Claim 2 fixed to a plate (10) comprising means (13) for holding the tubes (1, 2) relative to the detection means (3), the plate (10) being mounted pivotably on a support (11) in order to permit the positioning of the tube (1) of the first channel means in at least one position ranging from a horizontal position to a vertical position.

5. Detector according to one of the preceding claims, characterized in that the detection means (3) are optical detection means.

6. Detector according to one of the preceding claims, characterized in that it is used for the detection of the flow of a medical liquid in a circuit for an extracorporeal blood circulation.
